# EUROPEAN PATENT APPLICATION

(11) **EP 4 535 366 A1**
(43) Date of publication of application: **09.04.2025**
(21) Application number: 24729170.1
(22) Date of filing: 07.02.2024
(51) Int. Cl.: G16H 10/60

(54) **ELECTRONIC HEALTH RECORD SYSTEM**

(30) Priority: 21.08.2023 JP 2023133861
(71) Applicant: Iryou Jyouhou Gijyutu Kenkyusho Corporation, Yame-gun, Fukuoka 834-0115 (JP)
(72) Inventor: Himeno, Shinkichi, Yame-gun, Fukuoka 834-0102 (JP)
(74) Representative: dompatent von Kreisler Selting Werner - Partnerschaft von Patent- und Rechtsanwälten mbB
(86) International application number: PCT/JP2024/003984
(87) International publication number: WO 2025/041360

(57) **Abstract**

Provided is a system that supports creation of an order document and a record document by a medical practitioner in an electronic health record to support efficient medical activities by using a large language model that has learned a large amount of latest medical knowledge.

An electronic health record system used in combination with a large language model, the electronic health record system including: an electronic health record item extraction means configured to extract, from an electronic health record, information of an item necessary for processing of a document in an optionally designated document category; and a transcription means of an electronic health record content to a prompt configured to provide the extracted electronic health record content as a prompt of the large language model.

## Description

### Technical Field

The present invention relates to an electronic health record system with enhanced functionality using a large language model and with an improved user interface.

### Background Art

In medical settings or the like, a computer system (electronic health record) for efficiently recording medical instructions (orders) or records has been widely used. In addition, in recent years, the progress of artificial intelligence (AI) has been remarkable. In particular, large language models (LLMs) have attracted attention that enable a question and a response in natural language by learning a large amount of documents and data.

Related art documents related to this application are as follows.

### Citation List

### Patent Literature

Patent Literature 1: JP 2023-523644 A
Patent Literature 2: JP 2023-73095 A
Patent Literature 3: JP 5484317 B2
Patent Literature 4: JP 7313757 B2

### Summary of Invention

### Technical Problem

In implementation of medical care, examinations are planned on the basis of patient's symptoms and physical examination findings, a treatment plan is made according to the examination results, treatment is performed, and the record is kept. Since medical practitioners of various occupations such as doctors and nurses are involved, it goes without saying that electronic health records are useful for efficient medical activities. However, the progress of medical science is rapid, and new and highly effective medications and treatment methods are being developed one after another. Updating to the latest medical knowledge is a heavy burden on medical practitioners.

Arrangement of medical instructions (orders) such as examinations and treatments, implementation records, and the like are manually performed by medical practitioners. This requires a vast amount of work, resulting in long working hours. In addition, variations in therapeutic effects occur due to a difference in knowledge and skill levels among medical practitioners.

The present invention has been made to solve such conventional problems, and an object thereof is to provide a system that supports creation of an order document and a record document by a medical practitioner in an electronic health record to support efficient medical activities by using a large language model that has learned a large amount of latest medical knowledge.

### Solution to Problem

An electronic health record system according to claim 1, as a means for achieving the object, is an electronic health record system used in combination with a large language model, the electronic health record system including: an electronic health record item extraction means configured to extract, from an electronic health record, information of an item necessary for processing of a document in an optionally designated document category; and a transcription means of an electronic health record content to a prompt configured to provide the extracted electronic health record content as a prompt of the large language model, in which the electronic health record item extraction means includes an electronic health record extracted item management means for each document category configured to manage a list of electronic health record items necessary for each document category.

An electronic health record system according to claim 2 is the electronic health record system according to claim 1, including a transcription means of a response content to an electronic health record configured to transcribe, to the document of the electronic health record, a response content to the prompt.

An electronic health record system according to claim 3 is the electronic health record system according to claim 1 or 2, including an electronic health record additional document creation means configured to create at least one of an order document, a record document, or a disease name registration document to be added to the electronic health record concerning a response of the large language model.

An electronic health record system according to claim 4 is the electronic health record system according to claim 1 or 2, wherein the large language model includes a cooperation means with an external system.

An electronic health record system according to claim 5 is the electronic health record system according to claim 1 or 2, including a reference data limitation means configured to cause the large language model to additionally learn training data dedicated to medical care and refer only to the training data dedicated to medical care in generating a response.

An electronic health record system according to claim 6 is the electronic health record system according to claim 1 or 2, including a batch processing prompt creation means configured to collectively input a plurality of prompt contents to the prompt of the large language model.

An electronic health record system according to claim 7 is the electronic health record system according to claim 1 or 2, including a document content recommendation means configured to input patient information obtained by the electronic health record item extraction means as a prompt content for the document in the designated document category, and obtain a recommended content as a response in the large language model.

An electronic health record system according to claim 8 is the electronic health record system according to claim 1 or 2, wherein the electronic health record system includes an API for each electronic health record module, and the large language model calls the electronic health record module via the API to perform processing concerning necessary processing.

An electronic health record system according to claim 9 is the electronic health record system according to claim 8, including a series of processing groups management means configured to register the necessary processing as a series of collective processing groups so that the series of processing groups is designatable for the large language model.

### Advantageous Effects of Invention

The electronic health record system according to claim 1 includes the electronic health record item extraction means, so that the information of the item necessary for the processing of the document in the optionally designated document category is extracted from the electronic health record in the electronic health record system used in combination with the large language model.

Because the transcription means of an electronic health record content to a prompt is included, the extracted electronic health record content is provided as the prompt of the large language model.

The electronic health record system according to claim 1 includes the electronic health record extracted item management means for each document category, so that the list of electronic health record items necessary for each document category is managed in the electronic health record item extraction means.

The electronic health record system according to claim 2 includes the transcription means of a response content to an electronic health record, so that the response content to the prompt is transcribed to the document of the electronic health record.

The electronic health record system according to claim 3 includes the electronic health record additional document creation means, so that at least one of the order document, the record document, or the disease name registration document to be added to the electronic health record is created as necessary concerning the response of the large language model.

In the electronic health record system according to claim 4, the large language model includes the cooperation means with an external system.

Because the electronic health record system according to claim 5 includes the reference data limitation means, the large language model is subjected to reinforcement learning using the training data dedicated to medical care, and refers only to the training data dedicated to medical care in generating the response.

The electronic health record system according to claim 6 includes the batch processing prompt creation means, so that the plurality of prompt contents are collectively input to the prompt of the large language model.

The electronic health record system according to claim 7 includes the document content recommendation means, so that the patient information obtained by the electronic health record item extraction means is input as the prompt content for the document in the designated document category, and the recommended content is obtained as the response in the large language model.

In the electronic health record system according to claim 8, the electronic health record system includes the API for each electronic health record module, and the large language model calls the electronic health record module via the API to perform processing concerning the necessary processing.

Because the electronic health record system according to claim 9 includes the series of processing groups management means, the necessary processing is registered as the series of collective processing groups so that the series of processing groups is designatable for the large language model.

### Brief Description of Drawings

Fig. 1 is a hardware configuration diagram of the present invention.
Fig. 2 is an example of a user interface in a large language model.
Fig. 3 is a drawing illustrating an example of various document categories in an electronic health record.
Fig. 4 is an example in which the large language model is extended using an external cooperation system.
Fig. 5 is an example of data to be additionally learned by the large language model.
Fig. 6 is an example of correcting a medication prescription order in an electronic health record using the large language model.
Fig. 7 is a verification example of a disease name corresponding to a medication prescription order in an electronic health record.
Fig. 8 is an example of verifying a missing disease name and additionally registering the disease name in an electronic health record.
Fig. 9 is an explanatory diagram for referring to a history of blood tests and radiographic examinations in an electronic health record and making a recommended examination order.
Fig. 10 is a drawing illustrating a flow of referring to an electronic health record using the large language model, making a necessary additional order, correcting contents on the basis of the result, and recording the contents in the electronic health record.
Fig. 11 is a drawing of an electronic health record extracted item management means for each document category.
Fig. 12 is a drawing illustrating an example of a batch processing prompt.
Fig. 13 is a drawing illustrating an example in which symptoms, findings, examination findings, and a disease name are input to a prompt, and the LLM is caused to answer a recommended medication prescription example.
Fig. 14 is a conceptual diagram in which the LLM side mainly performs reference to an extracted item and creation of an order document for an examination and a treatment in an electronic health record.

### Description of Embodiments

An electronic health record system according to the present invention includes a server apparatus, a database, and a terminal.

The server apparatus is a known computer apparatus, and includes a computing device, a main storage device, an auxiliary storage device, an input device, an output device, and a communication device.

The computing device, the main storage device, the auxiliary storage device, the input device, the output device, and the communication device are connected to each other via an interface.

The computing device includes a known processor capable of executing a command set.

The main storage device includes a volatile memory such as a RAM capable of temporarily storing the command set.

The auxiliary storage device includes a non-volatile data storage capable of recording an OS and a program.

The data storage may be, for example, an HDD or an SSD.

The input device is, for example, a keyboard.

The output device is, for example, a display such as an LCD.

The communication device includes a network interface connectable to a network.

The server apparatus includes means such as an electronic health record item extraction means, a transcription means of an electronic health record content to a prompt, an electronic health record extracted item management means for each document category, a transcription means of a response content to an electronic health record, an electronic health record additional document creation means, a reference data limitation means, a batch processing prompt creation means, a document content recommendation means, and a series of processing groups management means.

The processor of the server apparatus exerts the operational effects of the means.

The database according to the present invention may be configured by the auxiliary storage device of the server apparatus, or may be configured by another auxiliary storage device independent of the server apparatus.

The database stores information handled by the electronic health record system.

The terminal according to the present invention has a hardware configuration of a known computer similarly to the server apparatus.

The server apparatus, the database, and the terminal according to the present invention can communicate via a network.

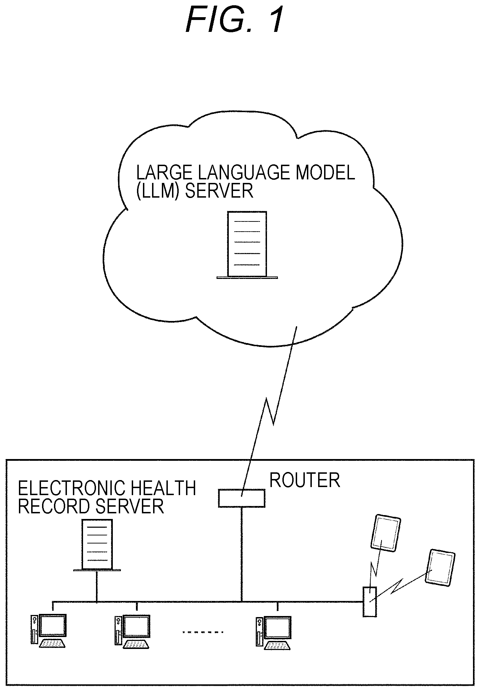

Fig. 1 is an example of a hardware configuration diagram of the present invention.

Because a large language model requires huge data and a large amount of computational resources, the large language model is usually on a cloud, and is connected to a local area network (LAN) in a medical institution via a router from an Internet line.

There is a server for electronic health records in the medical institution.

A staff member such as a doctor and a nurse uses the electronic health records and the large language model by using the terminal connected to the LAN.

Note that a part or all of the electronic health record system can be constructed on a cloud, and a part or all of a lightweight large language model can be installed in the medical institution.

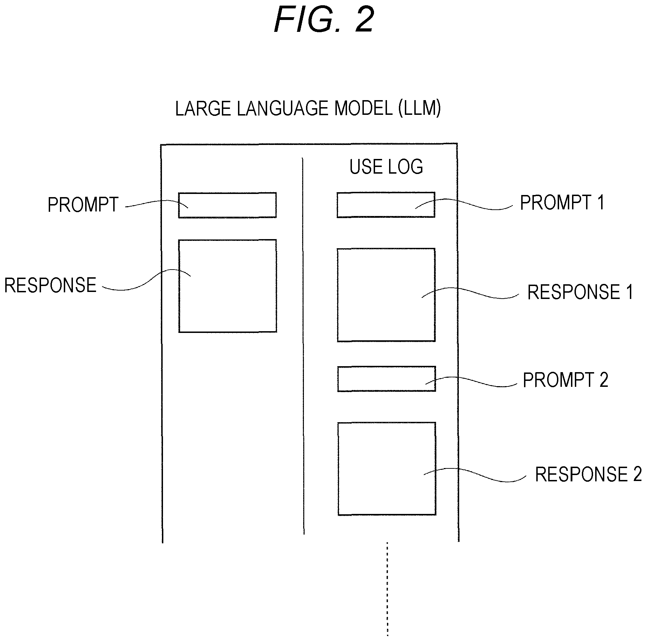

Fig. 2 is an example of a user interface in the large language model (LLM).

The LLM is currently being developed rapidly, and many models including ChatGPT (registered trademark of OpenAI), Bard, LaMDA (registered trademark of Google), and LLaMA (registered trademark of Meta Platforms) have been developed.

Of course, the user interfaces are different. However, as illustrated in Fig. 2, a standard user interface includes a frame for inputting a prompt for giving an instruction and asking a question to the LLM, a frame for displaying a response to the prompt, and at the same time, a frame for displaying a history of prompts and responses as a use log.

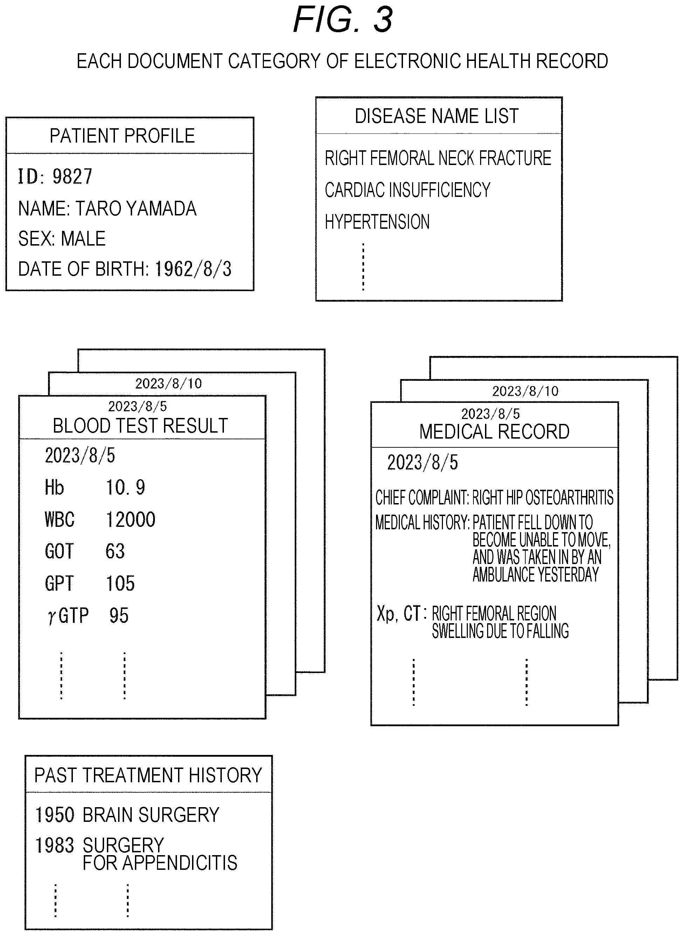

Fig. 3 illustrates an example of various document categories in an electronic health record.

In the electronic health record, various observation records and order documents for examinations, treatments, and the like for each patient, created by staff members of various occupations are recorded in time series.

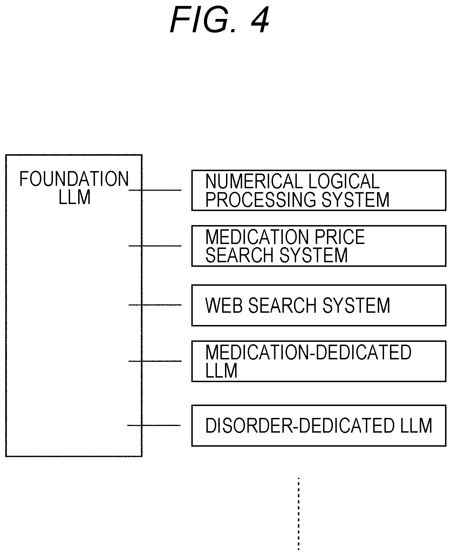

Fig. 4 is an extended example of the large language model by a dedicated LLM and Function Calling.

The LLM learns a large amount of data to configure a general-purpose language model (substrate trained model). However, knowledge in a specific field is not necessarily deep.

Therefore, the LLM is caused to intensively additionally learn knowledge in a specific field to configure a dedicated model (medication-dedicated LLM and below in the drawing).

In addition, it is known that the LLM of the general-purpose substrate trained model is weak against numerical calculation and logical operation.

To compensate for this weakness, there is a dedicated module, such as Wolfram Alpha (Non Patent Literature 1) (numerical logical processing system in the drawing), which responds to prompt questions from the LLM and return responses.

In addition, regarding recent events, time delay tends to occur in acquisition of knowledge.

Therefore, for example, it is conceivable to ask a WEB search engine about today's weather and acquire the response.

As one of methods for solving such a problem, Function Calling has been proposed.

This is to use another search engine, another database, or the function of an LLM specialized for a specific purpose via an application programming interface (API) from the LLM.

Taking the present invention as an example, the description of the efficacy of a medication is stable, but the medication price varies every year.

The current medication price information may be learned by the substrate LLM or the dedicated LLM. However, a quicker response is obtained by recording the current medication price information in a dedicated database and retrieving the current medication price information. In addition, the learning update frequency of the LLM can be reduced, and thus the cost can be reduced.

Moreover, when the dedicated LLM or the like can be used in parallel, the responding time can be expected to decrease.

Furthermore, by enabling information reference from the electronic health record system and writing to the electronic health record system as will be described later, the electronic health record system can be directly controlled from the LLM using the Function Calling.

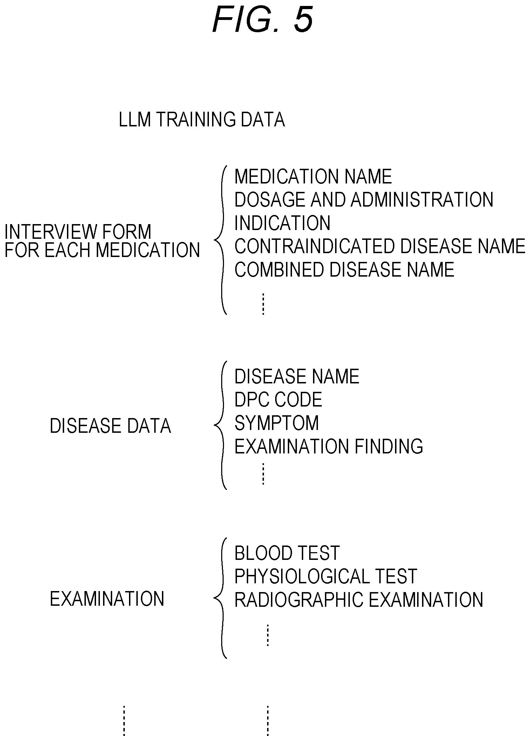

Fig. 5 is an example of data to be additionally learned by the large language model in the present invention.

In the interview form for each medication, for example, the medication name, dosage and administration, indications, and the like are described over several tens of pages.

These descriptions are based on data used for the approval of the Ministry of Health, Labor and Welfare.

The disease data includes medical textbooks, clinical practice guidelines for each disorder created by each learned society, and the like.

The examination includes a blood test, a physiological test such as an electrocardiogram, and an explanation book for X-ray, CT, or MRI, and the like. All of them are strictly supervised by professionals such as doctors and nurses, and are highly reliable.

In a substrate LLM, a large amount of training data includes some documents including malice or discrimination, and thus a malicious or discriminatory response is returned, which has been a problem.

In addition, "hallucinations" of generating a false response as if it were true also decrease the reliability of responses.

These problems can be avoided by limiting the range of training data to be referred to as a response to highly reliable training data as illustrated in this drawing (reference data limitation means).

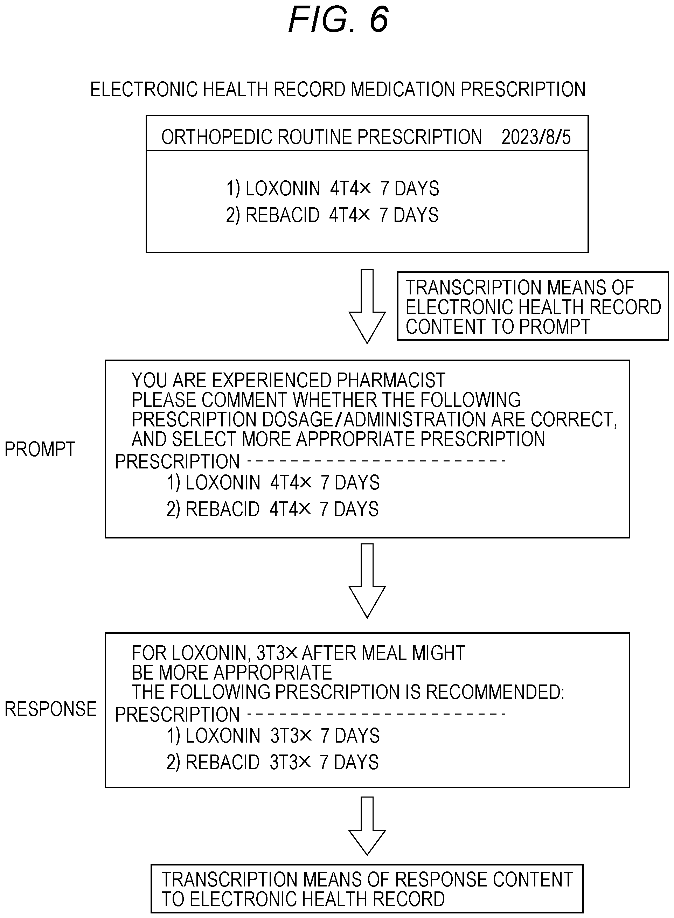

Fig. 6 is an example of correcting a medication prescription order in an electronic health record using the large language model.

Medication prescription is described in a medication prescription document of the electronic health record.

As illustrated in the drawing, the prescription contents are transcribed to a prompt of the LLM (transcription means of an electronic health record content to a prompt).

The LLM generates a response while referring to the description contents of the interview form or the like.

When a doctor determines that the correction contents are appropriate, the contents are transcribed to the medication prescription document of the electronic health record (transcription means of a response content to an electronic health record).

Note that "transcription to an electronic health record" includes transcription of the response contents to this document, of course, and also includes transcription of the response contents to a newly created document obtained by newly creating an additional document as necessary.

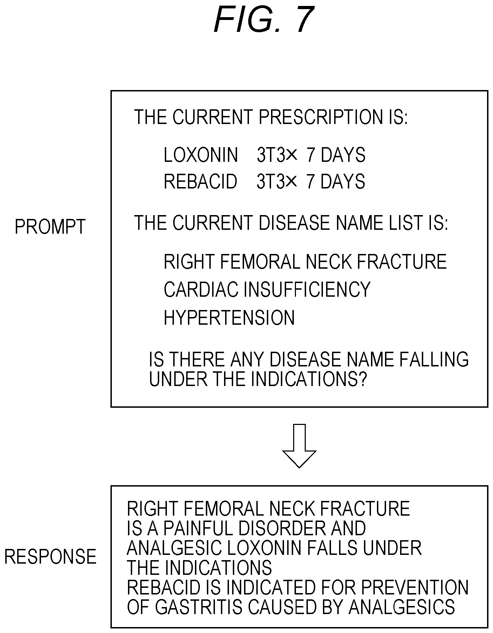

Fig. 7 is a verification example of a disease name corresponding to a medication prescription order in an electronic health record.

The medication prescription contents and the list of disease names of the electronic health record are extracted (electronic health record item extraction means), and transcribed to a prompt (transcription means of an electronic health record content to a prompt: aforementioned).

In a response, it is determined that there is no additional disease name by comparing the indication data of the medication and the indicated medications for each disorder.

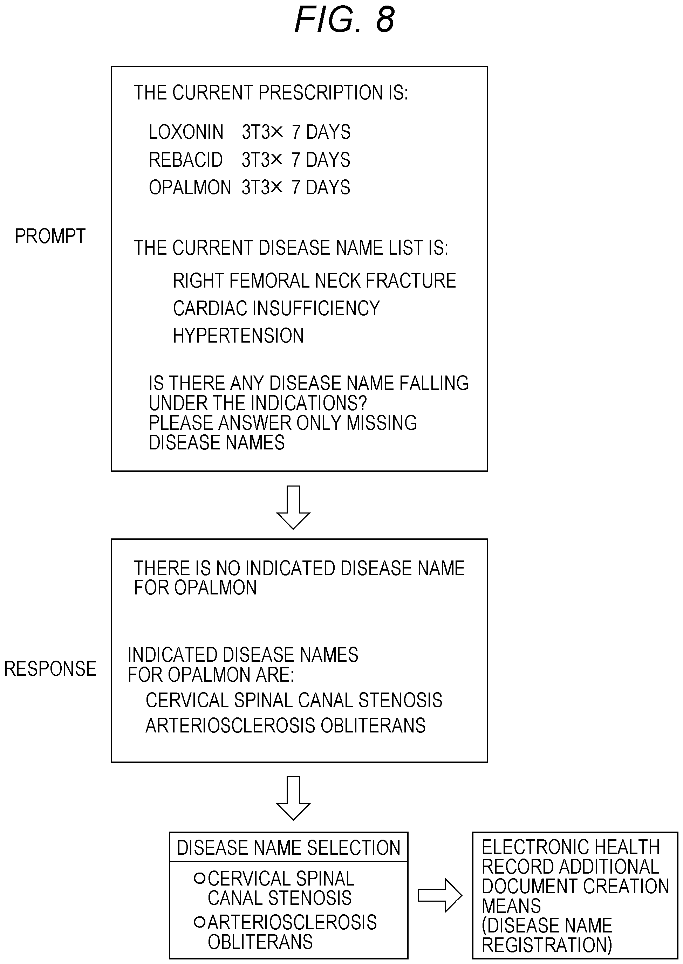

Fig. 8 is an example of verifying a missing disease name and additionally registering the disease name in an electronic health record.

A question is made with a prompt similar to that in Fig. 7.

In this drawing, it is pointed out that there is no indicated disease name for opalmon.

Furthermore, indicated disease names for opalmon are listed, a doctor is allowed to select a disease name, the disease name is transcribed to the electronic health record (transcription means of a response content to an electronic health record: aforementioned), and the disease name is registered.

As a result, it is possible to reduce assessments of medications and examinations (refusal of payment of medical service fees by an insurer) due to disease name omission, which sometimes exceeds several million yen per month.

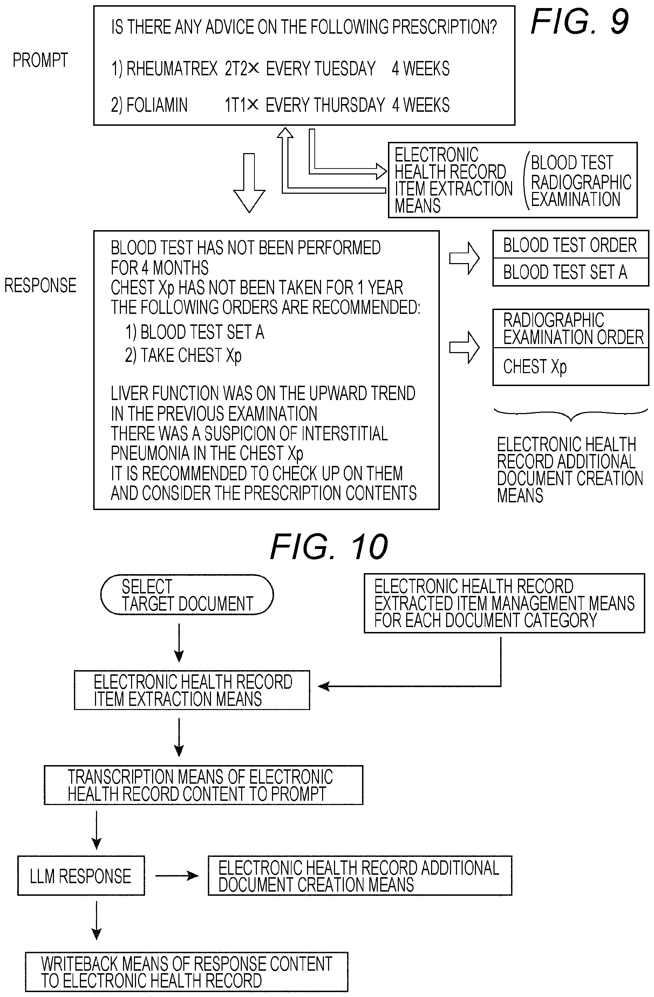

Fig. 9 is an explanatory diagram for referring to a history of blood tests and radiographic examinations in an electronic health record and recommending an examination order.

Medication prescription for rheumatoid arthritis is taken as an example.

The treatment guidelines for rheumatoid arthritis require checking the presence or absence of anemia, liver dysfunction, and renal dysfunction due to myelosuppression, which is a side effect of Rheumatrex, by a blood test at least once every several months.

In addition, it is recommended to take a chest XP about once every half year to check whether or not interstitial pneumonia, which is a side effect, is caused.

The electronic health record item extraction means extracts an implementation date for a recent blood test or chest Xp examination. The test or examination is recommended when the test or examination has not been performed for a while, and the order is created (electronic health record additional document creation means).

Here, when a side effect is observed to arise or have a deteriorating tendency, it is recommended to stop the medication.

Fig. 10 is a drawing illustrating a flow of referring to an electronic health record item using the large language model, making a necessary additional order, correcting contents on the basis of the result, and transcribing the contents to the electronic health record as illustrated in Figs. 6 to 9.

A target document (medication prescription in this example) is selected.

An electronic health record item necessary for LLM processing of the document is extracted from the electronic health record and transcribed to a prompt.

The electronic health record item necessary for the LLM processing is different in each document category.

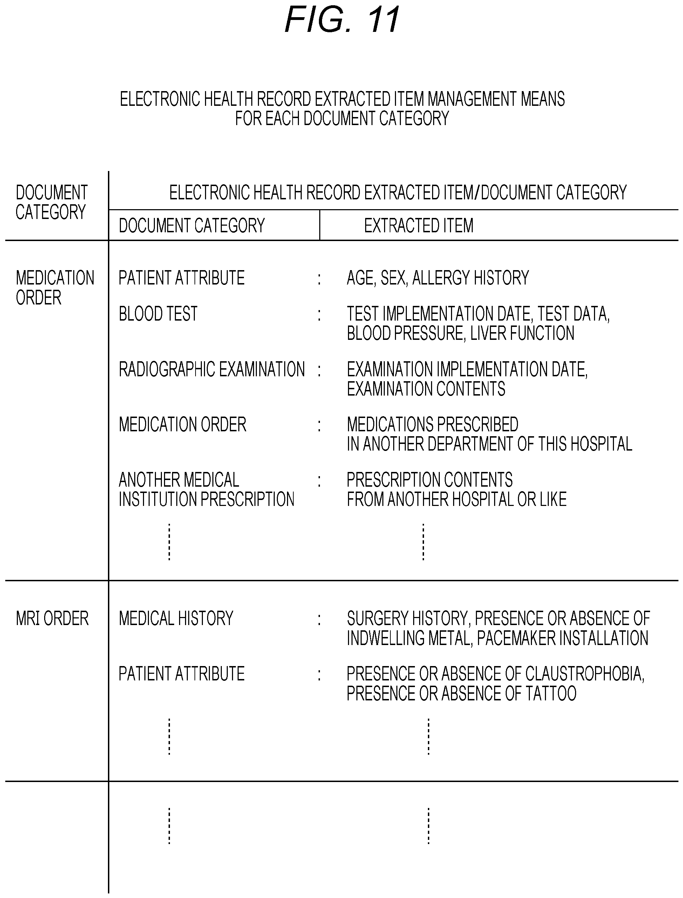

The transcription to the prompt may be manually performed each time. However, when electronic health record extracted items are registered for each document category as illustrated in Fig. 11 (electronic health record extracted item management means for each document category), the electronic health record extracted items are easily obtained without omission, and a necessary portion is transcribed to the prompt (transcription means of an electronic health record content to a prompt).

When a response is obtained from the LLM, the contents of the document are corrected (transcription means of a response content to an electronic health record), or an additional document such as a blood test order or a radiographic examination order is created as necessary (electronic health record additional document creation means).

Fig. 11 is a drawing for explaining the electronic health record extracted item management means for each document category.

Items to be transcribed to a prompt are managed for each document category in which the items are described.

As illustrated in Figs. 6 to 9, the extracted contents may be transcribed to a prompt as necessary.

In an MRI order, it is checked whether the item contents illustrated in the drawing are described, and if not described, the contents are confirmed by asking the person in question or a family member. When an intracranial aneurysm clip, pacemaker installation, or indwelling metal is near a target site, the examination is canceled.

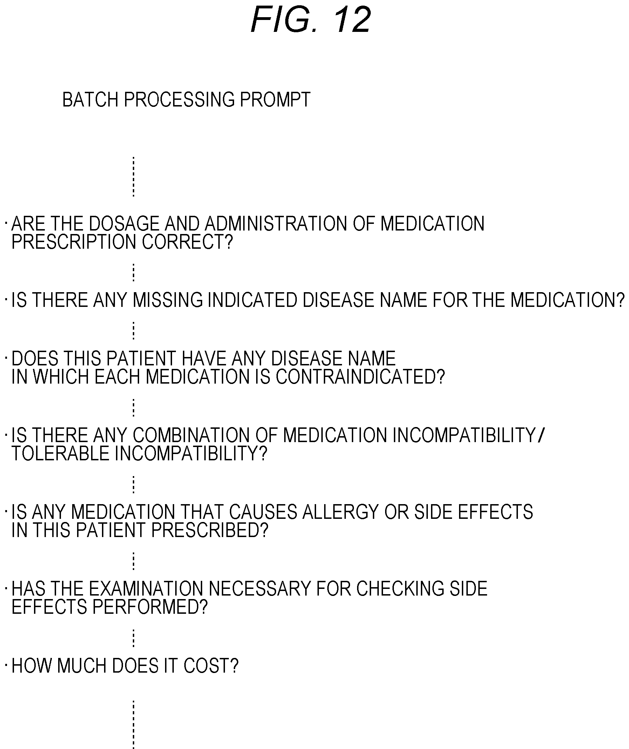

Fig. 12 is an example of a batch processing prompt.

In the above examples, the prompt and the response are generated one by one. However, in a case where there is a fixed processing routine, the processing requests of respective lines may be collectively generated in the prompt.

In a case where human intervention is needed during the processing, a decision may be requested or an additional document may be created, and the subsequent processing may be resumed.

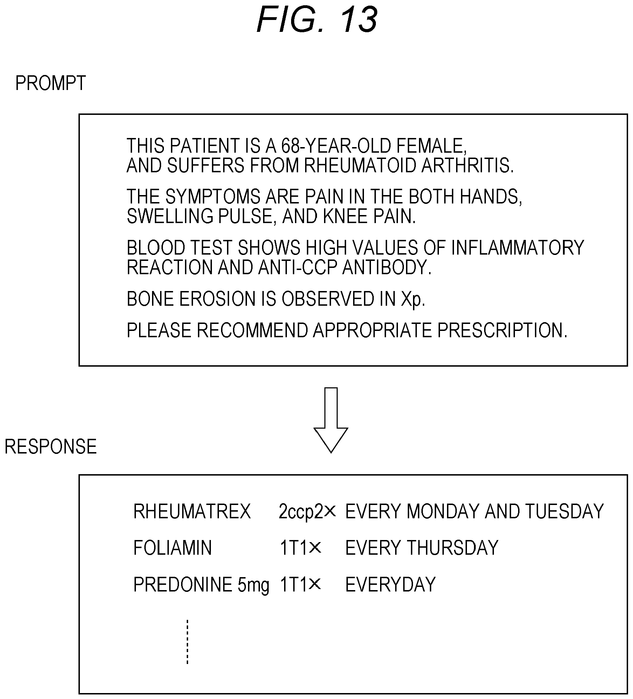

Fig. 13 is an example in which symptoms, findings, examination findings, and a disease name are input to a prompt, and the LLM is caused to answer a recommended medication prescription example.

In the above description, the description contents of the electronic health record are transcribed to the prompt and asked to the LLM. However, the LLM can also generate recommended contents as a generative AI.

In Fig. 13, the recommended medication prescription response is obtained from the attributes of a patient such as age and sex, the symptoms, the findings, the examination result data, and the disease name.

When a doctor determines that the response is appropriate, the response may be transcribed to the electronic health record.

If necessary, recommendations for a plurality of prescription time-series documents (so-called clinical path) including drip infusion, rehabilitation, lifestyle guidance, dietary recipe, and the like may be obtained and transcribed to the electronic health record.

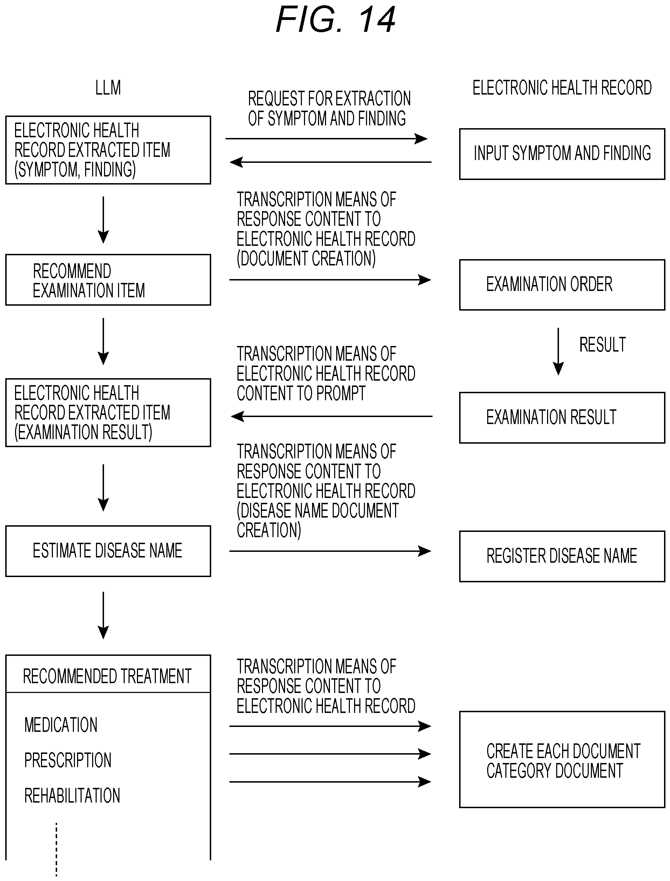

Fig. 14 is a conceptual diagram in which the LLM side mainly performs reference to an extracted item and creation of an order document for an examination and a treatment in an electronic health record.

In the examples of Figs. 6 to 12, the prompt is created on the basis of the document in the document category of the electronic health record, and the LLM is called.

That is, the category document side of the electronic health record mainly performs processing, and appropriately utilizes the LLM using the prompt.

On the other hand, in this drawing, the LLM side mainly calls the API of each module of the electronic health record, and performs data acquisition of items to be referred to such as symptom findings, creation of an examination order that is deemed necessary, estimation of a disease name and registration of the disease name in consideration of all the obtained examination results, and creation of an order document for treatments or examinations based on the estimated disease name as a series of operations.

By using this mechanism, it is possible to freely combine the electronic health record modules, and it is possible to easily cope with various pathological conditions.

Note that it is essential that each module of the electronic health record can be used from the LLM or other external systems via the API.

It is a matter of course that it is necessary to confirm the usage qualification in the API of each electronic health record module.

Although the embodiment has been described above, the specific configuration of the present invention is not limited to the above embodiment, and design changes and the like without departing from the gist of the invention are included in the present invention.

For example, although medical care has been described as an example, a similar system can be used in nursing care. Although ChatGPT and the like have been exemplified as the LLM, other systems can be similarly used, and a system newly developed in the future is also included in the present invention.

## Claims

1. An electronic health record system used in combination with a large language model, the electronic health record system comprising:
an electronic health record item extraction means configured to extract, from an electronic health record, information of an item necessary for processing of a document in an optionally designated document category; and
a transcription means of an electronic health record content to a prompt configured to provide the extracted electronic health record content as a prompt of the large language model, wherein
the electronic health record item extraction means includes an electronic health record extracted item management means for each document category configured to manage a list of electronic health record items necessary for each document category.

2. The electronic health record system according to claim 1, comprising a transcription means of a response content to an electronic health record configured to transcribe, to the document of the electronic health record, a response content to the prompt.

3. The electronic health record system according to claim 1 or 2, comprising an electronic health record additional document creation means configured to create at least one of an order document, a record document, or a disease name registration document to be added to the electronic health record concerning a response of the large language model.

4. The electronic health record system according to claim 1 or 2, wherein the large language model includes a cooperation means with an external system.

5. The electronic health record system according to claim 1 or 2, comprising a reference data limitation means configured to cause the large language model to additionally learn training data dedicated to medical care and refer only to the training data dedicated to medical care in generating a response.

6. The electronic health record system according to claim 1 or 2, comprising a batch processing prompt creation means configured to collectively input a plurality of prompt contents to the prompt of the large language model.

7. The electronic health record system according to claim 1 or 2, comprising a document content recommendation means configured to input patient information obtained by the electronic health record item extraction means as a prompt content for the document in the designated document category, and obtain a recommended content as a response in the large language model.

8. The electronic health record system according to claim 1 or 2, wherein the electronic health record system includes an API for each electronic health record module, and the large language model calls the electronic health record module via the API to perform processing concerning necessary processing.

9. The electronic health record system according to claim 8, comprising a series of processing groups management means configured to register the necessary processing as a series of collective processing groups so that the series of processing groups is designatable for the large language model.
